# EUROPEAN PATENT APPLICATION

(11) **EP 3 960 199 A1**
(43) Date of publication of application: **02.03.2022**
(21) Application number: 20795339.9
(22) Date of filing: 23.04.2020
(51) Int. Cl.: A61K 39/395, A61K 45/00, A61P 35/00, A61P 37/02, A61P 43/00, A61K 31/4709

(54) **IMMUNE CHECKPOINT INHIBITOR COMBINATION THERAPY USING QUINOLINE CARBOXAMIDE DERIVATIVE**

(30) Priority: 26.04.2019 JP 2019085041
(71) Applicant: Kabushiki Kaisha Yakult Honsha, Tokyo, 105-8660 (JP); General Incorporated Association Pharma Valley Project Supporting Organization, Mishima-shi, Shizuoka 411-0033 (JP)
(72) Inventor: ASAI, Akira, Shizuoka-shi, Shizuoka 422-8526 (JP); MIYOSHI, Nao, Shizuoka-shi, Shizuoka 422-8526 (JP); MURAOKA, Daisuke, Shizuoka-shi, Shizuoka 422-8526 (JP); OGO, Naohisa, Shizuoka-shi, Shizuoka 422-8526 (JP); TAKAHASHI, Hiroyuki, Tokyo 105-8660 (JP)
(74) Representative: Schiener, Jens
(86) International application number: PCT/JP2020/017522
(87) International publication number: WO 2020/218432

(57) **Abstract**

Provided is a method for using a STAT3 inhibitor which exhibits an excellent antitumor effect. An antitumor agent comprises a quinoline carboxamide derivative of formula (I) below or a salt thereof as an active ingredient, and is administered in combination with an immune checkpoint inhibitor. The antitumor agent is administered before the administration of the immune checkpoint inhibitor.

## Description

### Field of the Invention

The present invention relates to a combination therapy for cancer using a quinoline carboxamide derivative.

### Background of the Invention

STAT (signal transducers and activators of transcription), which is a transcription regulating factor, is a DNA-binding protein, and its activity is regulated by stimulations of various cytokines (e.g. IL-6 and interferons) or growth factors (e.g. EGF and PDGF). STAT activated by dimerization transfers into the nucleus, specifically recognizes a specific DNA sequence in a gene promotor region, and binds to the DNA sequence to induce transcription of many genes. That is, STAT is an essential mediator in a pathway for signaling from the cell surface to the nucleus, and is deeply involved in cell growth and differentiation.

It is known that STAT is classified into seven different members. Of these, STAT3 is expressed in most cell species, and constant activation and excessive expression of STAT3 are observed in cells of cancers such as lung cancer, skin cancer, pancreas cancer, ovary cancer, myeloma, breast cancer, prostate cancer, brain cancer, head and neck cancer, melanoma, leukemia lymphoma and multiple myeloma, and growth and infiltration of these cancer cells are considered to depend on STAT3.

Therefore, STAT3 may be useful as a target molecule for these cancers, and inhibitors of STAT3 are expected as anticancer agents. For example, specific quinoline carboxamide derivatives have been reported to have an excellent STAT3 inhibitory activity, and have antitumor activity against various cancers (Patent Literature 1).

On the other hand, cancer cells have various immune checkpoint molecules which hinder an immune response against cancer. Inhibition of the immune checkpoint is a novel treatment method for canceling an immunosuppressive mechanism to activate an immune reaction against cancer, and as immune checkpoint inhibitors, ipilimumab as an anti-CTLA-4 (cytotoxic T lymphocyte-associated antigen-4) antibody, nivolumab and pembrolizumab as anti-PD-1 (programmed cell death-1) antibodies, and the like have been already used domestically and internationally.

Patent Literature 1: JP-B-5650529

### Summary of the Invention

### Technical Problem

The present invention relates to providing a method for using a quinoline carboxamide derivative which exhibits an excellent antitumor effect.

### Solution to Problem

The present inventors extensively conducted studies in order to further enhance the usefulness of a quinoline carboxamide derivative of formula (I) below, and as a result, found that an excellent antitumor effect is obtained by combined administration in which the quinoline carboxamide derivative is orally administered in advance, and an immune checkpoint inhibitor is then administered.

That is, the present invention relates to the following 1) to 19).
1) An antitumor agent comprising a quinoline carboxamide derivative of formula (I) below or a salt thereof as an active ingredient, the antitumor agent being administered in combination with an immune checkpoint inhibitor, wherein the antitumor agent is administered before the administration of the immune checkpoint inhibitor.
2) An antitumor agent comprising a quinoline carboxamide derivative of formula (I) below or a salt thereof and an immune checkpoint inhibitor which are administered in combination, wherein the quinoline carboxamide derivative or a salt thereof is administered before the administration of the immune checkpoint inhibitor.
3) The antitumor agent according to 1) or 2), wherein the administration of the quinoline carboxamide derivative or a salt thereof is started one or more days before the administration of the immune checkpoint inhibitor.
4) The antitumor agent according to any one of 1) to 3), wherein the administration of the quinoline carboxamide derivative or a salt thereof is started two to seven days before the administration of the immune checkpoint inhibitor.
5) The antitumor agent according to any one of 1) to 4), wherein the route of administration of the quinoline carboxamide derivative or a salt thereof is oral administration.
6) The antitumor agent according to any one of 1) to 5), wherein R¹ and R² in formula (I) are the same or different, and each represent a substituted or unsubstituted aryl group or a substituted or unsubstituted aromatic heterocyclic group.
7) The antitumor agent according to 6), wherein at least one of the groups of R³, R⁴, R⁵ and R⁶ in formula (I) is a group other than a hydrogen atom.
8) The antitumor agent according to any one of 1) to 7), wherein in R¹ and R² in formula (I), the aryl group is a phenyl group and the aromatic heterocyclic group is a furyl group.
9) The antitumor agent according to any one of 1) to 7), wherein in formula (I), R¹ is a furyl group and R² is a substituted or unsubstituted phenyl group.
10) The antitumor agent according to any one of 1) to 9), wherein at least one of the groups of R³, R⁴, R⁵ and R⁶ is a trifluoromethoxy group.
11) The antitumor agent according to any one of 1) to 10), wherein R⁴ is a trifluoromethoxy group.
12) The antitumor agent according to any one of 1) to 11), wherein the quinoline carboxamide derivative of formula (I) is N-[5-(2-furyl)-1,3,4-oxadiazol-2-yl]-2-phenyl-6-trifluoromethoxy-4-quinolinecarboxamide.
13) The antitumor agent according to any one of 1) to 12), wherein the immune checkpoint inhibitor is a substance which suppresses a checkpoint function by acting on an immune checkpoint molecule selected from the group consisting of CTLA-4, PD-1, PD-L1, PD-L2, LAG-3, TIM3, BTLA, B7H3, B7H4, 2B4, CD160, A2aR, KIR, VISTA and TIGIT.
14) The antitumor agent according to any one of 1) to 12), wherein the immune checkpoint inhibitor is a PD-1 pathway inhibitor.
15) The antitumor agent according to 14), wherein the PD-1 pathway inhibitor is an anti-PD-1 antibody or an anti-PD-Ll antibody.
16) The antitumor agent according to any one of 1) to 15), wherein the cancer is one or more selected from the group consisting of non-small cell lung cancer, renal cell cancer, Hodgkin lymphoma, head and neck cancer, stomach cancer, malignant pleural mesothelioma, esophagus cancer, gastroesophageal cancer, small cell cancer, glioblastoma, urothelial cancer, muscle invasion urothelial cancer, urinary bladder cancer, non-muscle invasion urinary bladder cancer, bowel cancer, pancreas cancer, prostate cancer, Merkel cell carcinoma, thyroid gland cancer, hepatocyte cancer, breast cancer, ovary cancer, uterine body cancer, uterine cervical cancer, soft tissue sarcoma, virus-positive/negative solid cancer, central nerve-origin lymphoma/testis-origin lymphoma, MSI-High solid cancer, melanoma and leukemia.
17) Use of a quinoline carboxamide derivative of formula (I) below or a salt thereof for producing an antitumor agent which is administered in combination with an immune checkpoint inhibitor, the antitumor agent being administered before the administration of the immune checkpoint inhibitor.
18) A quinoline carboxamide derivative of formula (I) below or a salt thereof which is used in combination with an immune checkpoint inhibitor for treatment of cancer and administered before the administration of the immune checkpoint inhibitor.
19) A method for treating cancer, comprising administering an effective amount of a quinoline carboxamide derivative of formula (I) below or a salt thereof and an immune checkpoint inhibitor, wherein the quinoline carboxamide derivative or a salt thereof is administered before the administration of the immune checkpoint inhibitor. 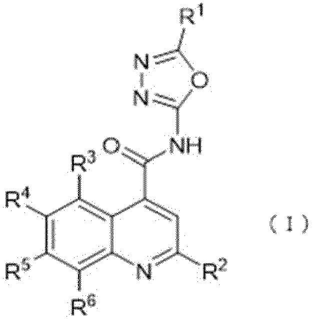
wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

### Advantageous Effect of the Invention

The antitumor agent according to the present invention enables cancer treatment which exhibits a high antitumor effect, and therefore a patient can live for a long term. The quinoline carboxamide derivative of the present invention can be orally administered and is effective for a wide range of cancer species, so that a combinatorial effect can be exhibited against any of cancers for which at least an immune checkpoint inhibitor is effective.

### Brief Description of the Drawings

[Figure 1] Figure 1 shows an antitumor effect of combined use of STX-1159 and an anti-PD-1 antibody.
[Figure 2] Figure 2 shows an antitumor effect of combined use of STX-1159 and an anti-PD-1 antibody.
[Figure 3] Figure 3 shows an antitumor effect of combined use of STX-1159 and an anti-PD-1 antibody.

### Detailed Description of the Invention

In a quinoline carboxamide derivative of formula (I) (hereinafter, also referred to as a "quinoline carboxamide derivative"), R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

Here, examples of the substituent in the alkyl group include a halogen atom, and a hydroxy group. The substituent in the aryl group or the aromatic heterocyclic group is appropriately selected from the group consisting of an alkyl group having 1 to 6 carbon atoms, an alkenyl group, an alkynyl group, a cycloalkyl group, an aralkyl group, OR^{a}, NR^{b}R^{c}, S(O)qR^{d} (wherein q is 0, 1 or 2), COR^{e}, COOR^{f}, OCOR^{g}, CONR^{h}Rⁱ, NR^{j}COR^{k}, NR^{l}COOR^{m}, NRⁿSO₂R^{o}, C(=NRP)NR^{q}R^{r}, NR^{s}SO₂NR^{t}R^{u}, SO₂NR^{v}R^{w}, a nitro group, a cyano group, a halogen atom and the like. Here, R^{a} to R^{w} may be the same or different, and each represent a hydrogen atom, an alkyl group, an alkenyl group, an alkynyl group, a cycloalkyl group or the like.

The number of substitutions with these substituents, which are the same or different, can be at most equal to the number of hydrogen atoms present in each group, and is preferably from 1 to 10, more preferably from 1 to 5.

Details of the groups specified in formula (I) above are described below. For groups having positional isomers in the groups, all possible positional isomers are shown.

Examples of the alkyl moiety of the alkyl group and the alkoxy group include linear or branched alkyl having 1 to 12 carbon atoms, specifically methyl, ethyl, propyl, isopropyl, butyl, isobutyl, sec-butyl, tert-butyl, pentyl, isopentyl, neopentyl, hexyl, heptyl, octyl, nonyl, decyl, undecyl and dodecyl.

Examples of the cycloalkyl group include three- to twelve-membered cycloalkyl groups which are saturated or optionally have partially unsaturated bonds. The cycloalkyl group may be a monocyclic cycloalkyl group, or a polycyclic condensed cycloalkyl group in which a plurality of the monocyclic cycloalkyl groups are condensed together, or the monocyclic cycloalkyl group is condensed with an aryl group or an aromatic heterocyclic group. Examples of the monocyclic cycloalkyl group include monocyclic cycloalkyl having 3 to 8 carbon atoms, specifically cyclopropyl, cyclobutyl, cyclopentyl, cyclohexyl, cycloheptyl, cyclooctyl, cyclododecyl and 1-cyclohexenyl. Examples of the polycyclic cycloalkyl group include polycyclic cycloalkyl having 5 to 12 carbon atoms, specifically pinanyl, adamantyl, bicyclo[3.3.1]octyl and bicyclo[3.1.1]heptyl.

Examples of the alkenyl group include linear or branched alkenyl having 2 to 12 carbon atoms, specifically vinyl, allyl, 1-propenyl, isopropenyl, methacryl, butenyl, 1,3-butadienyl, crotyl, pentenyl, hexenyl, heptenyl, decenyl and dodecenyl.

Examples of the alkynyl group include linear or branched alkynyl having 2 to 12 carbon atoms, specifically ethynyl, propargyl, 1-propynyl, isopropynyl, 2-butynyl, pentynyl, 2-penten-4-ynyl, hexynyl, heptynyl, decynyl and dodecynyl.

Examples of the aryl group include aryl having 6 to 14 carbon atoms, specifically phenyl, naphthyl, anthryl and phenanthryl.

Example of the aromatic heterocyclic group includes a five- or six-membered aromatic heterocyclic group containing at least one hetero atoms, for example nitrogen, oxygen or sulfur which are the same or different and the heterocyclic group may be a monocyclic heterocyclic group, or a polycyclic condensed aromatic heterocyclic group in which a plurality of the monocyclic heterocyclic groups are condensed together, or the monocyclic heterocyclic group is condensed with an aryl group, for example a dicyclic or tricyclic heterocyclic group. Specific examples of the monocyclic aromatic heterocyclic group include furyl, thienyl, pyrrolyl, imidazolyl, pyrazolyl, triazolyl, tetrazolyl, oxazolyl, isoxazolyl, oxadiazolyl, thiazolyl, thiadiazolyl, isothiazolyl, pyridyl, pyrimidinyl, pyrazinyl, pyridazinyl and triazinyl. Examples of the polycyclic condensed aromatic heterocyclic group include benzofuryl, benzothienyl, indolyl, isoindolyl, indazolyl, benzoimidazolyl, benzotriazolyl, benzooxazolyl, benzothiazolyl, carbazolyl, purinyl, quinolyl, isoquinolyl, quinazolinyl, phthalazinyl, quinoxalinyl, cinnolinyl, naphthyridinyl, pyridopyrimidinyl, pyrimidopyrimidinyl, pteridinyl, acridinyl, thianthrenyl, phenoxathinyl, phenoxazinyl, phenothiazinyl and phenazinyl.

Examples of halogen atoms include atoms of fluorine, chlorine, bromine and iodine.

Of the quinoline carboxamide derivatives of the present invention, compounds are preferable in which R¹ and R² are the same or different, and each represent a substituted or unsubstituted aryl group, or a substituted or unsubstituted aromatic heterocyclic group. A phenyl group, a naphthyl group or the like is suitably exemplified as the aryl group, and a furyl group, a thienyl group or the like is suitably exemplified aromatic heterocyclic group. The quinoline carboxamide derivative is more preferably a compound in which R¹ represents a furyl group, and R² represents a substituted or unsubstituted phenyl group, a substituted or unsubstituted furyl group or a substituted or unsubstituted thienyl group. Preferable examples of the substituent in the substituted phenyl group include an alkyl group such as a methyl group, a substituted or unsubstituted alkoxy group such as a methoxy group or a difluoromethoxy group, a halogen atom such as a fluorine atom or a chlorine atom, a hydroxy group, an alkoxycarbonyl group such as a tert-butoxycarbonyl group, an amino group, a nitro group, and a cyano group, and preferable examples of the substituent in the substituted furyl group and the substituted thienyl group include an alkyl group such as a methyl group and a halogen atom such as a chlorine atom.

The quinoline carboxamide derivative is even more preferably a compound in which R³, R⁵ and R⁶ each represent a hydrogen atom, and R⁴ represents OR⁸ (preferably a methoxy group or a trifluoromethoxy group).

As specific examples of the quinoline carboxamide derivative of the present invention, for example, N-[5-(2-furyl)-1,3,4-oxaziazol-2-yl]-2-phenyl-4-quinoline carboxamide, N-[5-(3-furyl)-1,3,4-oxadiazol-2-yl]-2-phenyl-4-quinoline carboxamide, 2-phenyl-N-(5-phenyl-1,3,4-oxadiazol-2-yl)-4-quinoline carboxamide, N-[5-(4-chlorophenyl)-1,3,4-oxadiazol-2-yl]-2-phenyl-4-quinoline carboxamide, N-[5-(4-nitrophenyl)-1,3,4-oxadiazol-2-yl]-2-phenyl-4-quinoline carboxamide, 2-phenyl-N-[5-(3-pyridyl)-1,3,4-oxadiazol-2-yl]-4-quinoline carboxamide, N-[5-(2-furyl)-1,3,4-oxadiazol-2-yl]-2-(3-nitrophenyl)-4-quinoline carboxamide, 2-(4-cyanophenyl)-N-[5-(2-furyl)-1,3,4-oxadiazol-2-yl]-4-quinoline carboxamide, 2-(2-furyl)-N-[5-(2-furyl)-1,3,4-oxadiazol-2-yl]-4-quinoline carboxamide, 2-(5-chloro-2-thienyl)-N-[5-(2-furyl)-1,3,4-oxadiazol-2-yl]-4-quinoline carboxamide, N-[5-(2-furyl)-1,3,4-oxadiazol-2-yl]-6-methoxy-2-phenyl-4-quinoline carboxamide, 2-(1-butoxy)-N-[5-(2-furyl)-1,3,4-oxadiazol-2-yl]-4-quinoline carboxamide, 2-(2-chlorophenyl)-N-[5-(2-furyl)-1,3,4-oxadiazol-2-yl]-4-quinoline carboxamide, N-[5-(2-furyl)-1,3,4-oxadiazol-2-yl]-2-(2-hydroxyphenyl)-4-quinoline carboxamide, 2-(2-aminophenyl)-N-[5-(2-furyl)-1,3,4-oxadiazol-2-yl]-4-quinoline carboxamide, 2-(3-chlorophenyl)-N-[5-(2-furyl)-1,3,4-oxadiazol-2-yl]-4-quinoline carboxamide, N-[5-(2-furyl)-1,3,4-oxadiazol-2-yl]-2-(3-methoxyphenyl]-4-quinoline carboxamide, 2-(3-cianophenyl)-N-[5-(2-furyl)-1,3,4-oxadiazol-2-yl]-4-quinoline carboxamide, 2-(3-tert-butoxycarbonylphenyl)-N-[5-(2-furyl)-1,3,4-oxadiazol-2-yl]-4-quinoline carboxamide, 2-(4-fluorophenyl)-N-[5-(2-furyl)-1,3,4-oxadiazol-2-yl]-4-quinoline carboxamide, 2-(4-chlorophenyl)-N-[5-(2-furyl)-1,3,4-oxadiazol-2-yl]-4-quinoline carboxamide, N-[5-(2-furyl)-1,3,4-oxadiazol-2-yl]-2-(4-methylphenyl)-4-quinoline carboxamide, 2-(4-difluoromethoxyphenyl]-N-[5-(2-furyl)-1,3,4-oxadiazol-2-yl]-4-quinoline carboxamide, N-[5-(2-furyl)-1,3,4-oxadiazol-2-yl]-2-(4-hydroxyphenyl)-4-quinoline carboxamide, N-[5-(2-furyl)-1,3,4-oxadiazol-2-yl]-2-(4-methoxyphenyl)-4-quinoline carboxamide, N-[5-(2-furyl)-1,3,4-oxadiazol-2-yl]-2-(4-nitrophenyl)-4-quinoline carboxamide, 2-(4-tert-butoxycarbophenyl)-N-[5-(2-furyl)-1,3,4-oxadiazol-2-yl]-4-quinoline carboxamide, N-[5-(2-furyl)-1,3,4-oxadiazol-2-yl]-2-(2,4-dimethylphenyl)-4-quinoline carboxamide, N-[5-(2-furyl)-1,3,4-oxadiazol-2-yl]-2-(3,4-dimethoxyphenyl)-4-quinoline carboxamide, N-[5-(2-furyl)-1,3,4-oxadiazol-2-yl]-2-(3,4-methylenedioxyphenyl)-4-quinoline carboxamide, N-[5-(2-furyl)-1,3,4-oxadiazol-2-yl]-2-(1-naphthyl)-4-quinoline carboxamide, N-[5-(2-furyl)-1,3,4-oxadiazol-2-yl]-2-(6-methoxy-2-naphthyl)-quinoline carboxamide, N-[5-(2-furyl)-1,3,4-oxadiazol-2-yl]-2-(5-methyl-2-furyl)-4-quinoline carboxamide, N-[5-(2-furyl)-1,3,4-oxadiazol-2-yl]-2-phenyl-6-trifluoromethoxy-4-quinoline carboxamide, N-[5-(5-nitro-2-furyl)-1,3,4-oxadiazol-2-yl]-2-phenyl-4-quinoline carboxamide, N-[5-(2-furyl)-1,3,4-oxadiazol-2-yl]-6-(4-hydroxyphenyl)-2-phenyl-4-quinoline carboxamide, N-[5-(2-furyl)-1,3,4-oxadiazol-2-yl]-6-(3-thienyl)-2-phenyl-4-quinoline carboxamide, N-[5-(2-furyl)-1,3,4-oxadiazol-2-yl]-6-(3-pyridyl)-2-phenyl-4-quinoline carboxamide, N-[5-(2-furyl)-1,3,4-oxadiazol-2-yl]-2-phenyl-6-trifluoromethoxy-4-quinoline carboxamide, N-[5-(2-chlorophenyl)-1,3,4-oxadiazol-2-yl]-2-phenyl-6-trifluoromethoxy-4-quinoline carboxamide, N-[5-(4-methoxyphenyl)-1,3,4-oxadiazol-2-yl]-2-phenyl-6-trifluoromethoxy-4-quinoline carboxamide, N-[5-(5-chloro-2-thienyl)-1,3,4-oxadiazol-2-yl]-2-phenyl-4-quinoline carboxamide, N-[5-(4-methoxyphenyl)-1,3,4-oxadiazol-2-yl]-2-phenyl-4-quinoline carboxamide and N-(5-phenyl-1,3,4-oxadiazol-2-yl)-2-(2-thienyl)-4-quinoline carboxamide are preferable, and N-[5-(2-furyl)-1,3,4-oxadiazol-2-yl]-2-phenyl-6-trifluoeomethoxy-4-quinoline carboxamide is more preferable.

Examples of the salt of the quinoline carboxamide derivative according to the present invention include pharmacologically acceptable acid addition salts, metal salts, ammonium salts, organic amine addition salts and amino acid addition salts. Examples of the pharmacologically acceptable acid addition salt include salts of inorganic acids such as hydrochloric acid, hydrobromic acid, sulfuric acid, nitric acid, phosphoric acid and boric acid; and organic acids such as carboxylic acids such as formic acid, acetic acid, propionic acid, fumaric acid, malonic acid, succinic acid, maleic acid, tartaric acid, citric acid and benzoic acid, sulfonic acids such as methanesulfonic acid and p-toluenesulfonic acid, and amino acids such as glutamic acid and aspartic acid. Examples of the pharmacologically acceptable metal salt include salts of alkali metals such as lithium, sodium and potassium; salts of alkali earth metals such as magnesium and calcium; and metals such as aluminum and zinc, examples of the pharmacologically acceptable ammonium salt include salts of ammonium, tetramethylammonium and the like, examples of the pharmacologically acceptable organic amine salt include salts of triethylamine, piperidine, morpholine, toluidine and the like, and examples of the pharmacologically acceptable amino acid addition salt include addition salts of lysine, glycine, phenylalanine and the like.

A quinoline carboxamide derivative or a salt thereof according to the present invention is disclosed as a STAT3 inhibitor in JP-B-5650529 (Patent Literature 1), and can be manufactured by the method described in this publication. The quinoline carboxamide derivative or a salt thereof is known to exhibit an antitumor effect by inhibiting formation of a dimer of STAT3.

An immune checkpoint inhibitor for use in the present invention means a substance which can suppress a checkpoint function by acting on an immune checkpoint molecule which is a molecule transmitting a suppressive co-signal to exhibit an immunosuppressive function. Examples of the immune checkpoint molecule include PD-1, CTLA-4, OX-40, TIM3 (T cell immunoglobulin and mucin-3), LAG-3 (Lymphocyte activation gene 3), VISTA(V-domain Ig-containing suppressor of T cell activation), GITR (Glucocorticoid-induced TNFR-related protein), 4-1BB, CD40, ICOS, CD28, TIGIT (T cell immunoglobulin and ITIM domain), BTLA (B and T lympho-cyte attenuator), CD160, PD-L1 (programmed cell death-ligand 1), PD-L2 (programmed cell death-ligand 2), CD86, OX40L, Galectin-9/HMGB1, GITRL, 4-1BBL, CD40L, ICOSL, CD80, CD112/CD155, HVEM, B7H3, B7H4, 2B4, A2aR (adenosine A2a receptor), KIR (killer inhibitory receptor).

The immune checkpoint inhibitor is preferably an inhibitor of human immune checkpoint molecules, more preferably a neutralizing antibody against human checkpoint molecules.

Examples of the immune checkpoint inhibitor include anti-CTLA-4 antibodies (e.g. Ipilimumab (YERVOY (registered trademark) and Tremelimumab AGEN-1884)); anti-PD-1 antibodies (e.g. Nivolumab (OPDIVO (registered trademark)), REGN-2810, Pembrolizumab (KEYTRUDA (registered trademark)), PDR-001, BGB-A317, AMP-514 (MEDIO 680), BCD-100, IBI-308, JS-001, PF-06801591 and TSR-042); anti-PD-Ll antibodies (e.g. Atezolizumab (TECENTRIQ (registered trademark), RG7446 and MPDL 3280A), Avelumab (BAVENCIO (registered trademark), PF-06834635, MSB0010718C), Durvalumab (MEDI4736), BMS-936559, CA-170, LY-3300054, FAZ053); anti-PD-L2 antibodies (e.g. rHIgM12B7); PD-1 inhibitors (e.g. AUNP-12); PD-L1 fusion proteins; PD-L2 fusion proteins (e.g. AMP-224); anti-Tim-3 antibodies (e.g. MBG 453); anti-LAG-3 antibodies (e.g. BMS-986016 and LAG 525); anti-KIR antibodies (e.g. Lirilumab); anti-OX-40 antibodies (e.g. GSK 3174998 and PF-04518600); anti-VISTA antibodies (e.g. IGN-381); anti-GITR antibodies (BMS-986156); and anti-4-1BB antibodies (e.g. Utomilumab).

The immune checkpoint inhibitor according to the present invention also encompasses antibodies including heavy chain and light chain complementarity determining regions (CDRs) or a variable region (VR) of any of the above-described known antibodies. The antibody may be a functional antibody fragment, and examples of the antibody fragment include Fab, Fab', F(ab')2, Fv, scFv and dsFv.

As the immune checkpoint inhibitor according to the present invention, PD-1 pathway inhibitors which inhibit PD-1/PD-1 ligand pathways, such as anti-PD-1 antibodies, anti-PD-Ll antibodies, anti-PD-L2 antibodies, PD-1 inhibitors, PD-L1 fusion proteins and PD-L2 fusion proteins; and anti-CTLA-4 antibodies are preferable, PD-1 pathway inhibitors are more preferable, anti-PD-1 antibodies, anti-PD-Ll antibodies and anti-PD-L2 antibodies are more preferable, and anti-PD-1 antibodies are especially preferable.

When a quinoline carboxamide derivative or a salt thereof in the present invention and an immune checkpoint inhibitor are administered in combination, a markedly excellent antitumor effect is exhibited by administering the quinoline carboxamide derivative or a salt thereof before the administration of the immune checkpoint inhibitor as in Examples described later.

This may be because by administering the quinoline carboxamide derivative or a salt thereof in advance, a tumor immune environment can be adjusted to a state favorable for the immune checkpoint inhibitor to exhibit an effect, i.e. a state in which tumor immune cells are activated, a state in which an immunosuppressive environment in tumor is cancelled, or the like. Therefore, even for a tumor for which a sufficient antitumor effect of immune reaction cannot be obtained by administration of the immune checkpoint inhibitor, an antitumor effect of the immune checkpoint inhibitor can be expected by administering the quinoline carboxamide derivative in advance to adjust the tumor immune environment to a favorable state.

Therefore, using a quinoline carboxamide derivative or a salt thereof in the present invention in combination with an immune checkpoint inhibitor and administering the quinoline carboxamide derivative or a salt thereof before the administration of the immune checkpoint inhibitor provides an effective method for treatment of cancer, and a quinoline carboxamide derivative or a salt thereof in the present invention can be an antitumor agent which is administered in combination with an immune checkpoint inhibitor, the antitumor agent being administered before the administration of the immune checkpoint inhibitor. A quinoline carboxamide derivative or a salt thereof in the present invention can be used for producing an antitumor agent which is administered in combination with an immune checkpoint inhibitor, the antitumor agent being administered before the administration of the immune checkpoint inhibitor.

The "combined administration" means that a quinoline carboxamide derivative or a salt thereof in the present invention and an immune checkpoint inhibitor are administered in combination within a certain period, and in the present invention, the order of administration thereof is required to be such that the administration of the quinoline carboxamide derivative or a salt thereof precedes the administration of the immune checkpoint inhibitor. Preferably, the administration of the quinoline carboxamide derivative or a salt thereof is started one or more days before the administration of the immune checkpoint inhibitor.

The time of starting the administration of a quinoline carboxamide derivative or a salt thereof in the present invention is more preferably from two or more days before the administration of the immune checkpoint inhibitor, more preferably from two to twenty eight days before the administration of the immune checkpoint inhibitor, more preferably from two to twenty one days before the administration of the immune checkpoint inhibitor, more preferably from two to fourteen days before the administration of the immune checkpoint inhibitor, more preferably from two to seven days before the administration of the immune checkpoint inhibitor.

The administration of the quinoline carboxamide or a salt thereof according to the present invention may be started before the administration of the immune checkpoint inhibitor, may be continued after the start of administering the immune checkpoint inhibitor, or may be ended or halted before the start of administering the immune checkpoint inhibitor.

The cancer which can be treated by the antitumor agent of the present invention is not particularly limited, and examples thereof include malignant neoplasms; carcinomas, undifferentiated cancer; giant cell and spindle cell cancer; small cell cancer; papillary cancer; squamous cancer; lymphoepithelial cancer; basal cell cancer; calcifying epithelial cancer; transitional cell cancer; papillary transitional cell cancer; adenocarcinoma; malignant gastrinoma; bile duct cancer; hepatocyte cancer; combined hepatocyte cancer and bile duct cancer; trabecular adenocarcinoma; adenoid cystic cancer; adenocarcinoma in adenomatous polyp; familial polyposis coli; solid cancer; malignant carcinoid tumor; branchiolo-alveolar adenocarcinoma; papillary adenocarcinoma; chromophobic cancer; eosinophilic cancer: acidophilic adenocarcinoma; basophilic cancer; clear cell adenocarcinoma; granular cell cancer; follicular cancer; papillary and follicular cancer; non-encapsulated sclerosing cancer; adrenal cortex cancer; endometroid carcinoma; skin appendage cancer; apocrine adenocarcinoma; sebaceous adenocarcinoma; ceruminous adenocarcinoma; mucoepidermoid cancer; cystadenocarcinoma; papillary cystadenocarcinoma; papillary serous cystadenocarcinoma; mucinous cystadenocarcinoma; mucinous adenocarcinoma; signet-ring cell cancer; infiltrating duct cancer; medullary cancer: lobular cancer; inflammatory cancer; breast Paget disease; acinic cell cancer; adenosquamous cancer; adenocarcinoma with squamous metaplasia; malignant thymoma; malignant ovarian stroma tumor; malignant thecal tumor; malignant granulosa cell tumor; malignant roblastoma; Sertoli cell cancer; malignant Leydig cell tumor; malignant lipid cell tumor; malignant paraganglioma; malignant extramammary paraganglioma; phenochromocytoma; glomus cell tumor; malignant melanoma; amelanotic melanoma; superficial spreading melanoma; malignant melanoma in giant pigmented nevus; epithelioid cell melanoma; malignant blue nevus; sarcoma; malignant fibrous histiocytoma; myxosarcoma; liposarcoma; leiomyosarcoma; rhabdomyosarcoma; embryonic rhabdomyosarcoma; ovarian rhabdomyosarcoma; stromal sarcoma; malignant mixed tumor; Mullerian mixed tumor; nephroblastoma; hepatoblastoma; carcinosarcoma; malignant mesenchymoma; malignant Brenner tumor: malignant phyllodes tumor; synovial tumor; malignant mesothelial tumor; dysgerminoma; embryonic cancer; malignant teratoma; malignant ovarian struma; chorionic cancer; malignant mesonephroma; hemangiosarcoma; malignant hemangioendothelioma; Kaposi sarcoma; malignant perithelioma; lymphangiosarcoma; osteosarcoma; juxtacortical osteosarcoma; chondrosarcoma; malignant chondroblastoma; mesenchymal chondrosarcoma; giant cell tumor of bone; Ewing's sarcoma; malignant odontogenic tumor; ameloblastic odontosarcoma; malignant ameloblastoma; ameloblastic fibrosarcoma; malignant pinealoma; chordoma; malignant glioma; ependymoma; astrocytoma; protoplasmic astrocytoma; fibrous astrocytoma; astroblastoma; glioblastoma; oligodendroglioma; oligodendroblastoma; primitive neuroectodermal tumor; cerebellar sarcoma; ganglioneuroblastoma; neuroblastoma; retinoblastoma; olfactory neurogenetic tumor; malignant meningioma; neurofibrosarcoma; malignant neurilemmoma; malignant granular cell tumor; malignant lymphoma; Hodgkin disease; Hodgkin lymphoma; paragranuloma; malignant small lymphocytic lymphoma; malignant diffuse large-cell lymphoma; malignant follicular lymphoma; mycosis fungoides; other specified non-Hodgkin's lymphoma; malignant histiocytosis; multiple myeloma; mast cell sarcoma; immunoproliferative small intestine disease; leukemia; lymphatic leukemia; plasma cell leukemia; erythroid leukemia; lymphosarcoma cell leukemia; myelocytic leukemia; basophilic leukemia; eosinophilic leukemia; monocytic leukemia; mast cell leukemia; megakaryoblastic leukemia; myelogenous sarcoma; and hairy cell leukemia, and non-small cell lung cancer, renal cell cancer, Hodgkin lymphoma, head and neck cancer, stomach cancer, malignant pleural mesothelioma, esophagus cancer, gastroesophageal cancer, small cell cancer, glioblastoma, urothelial cancer, muscle invasion urothelial cancer, urinary bladder cancer, non-muscle invasion urinary bladder cancer, bowel cancer, pancreas cancer, prostate cancer, Merkel cell carcinoma, thyroid gland cancer, hepatocyte cancer, breast cancer, ovary cancer, uterine body cancer, uterine cervical cancer, soft tissue sarcoma, virus-positive/negative solid cancer, central nerve-origin lymphoma/testis-origin lymphoma, MSI-High solid cancer, melanoma and leukemia are preferable.

The quinoline carboxamide derivative or a salt thereof of the present invention can be orally administered because of being non-peptidic and non-nucleoside, and can effectively exhibit the above-mentioned combinatorial effect through oral administration. The advantage of oral administration is high convenience for patients because the oral administration is not invasive and enables outpatient treatment. For example, oligonucleotide is not absorbed and therefore does not exhibit an effect in oral administration, and is very difficult to subject to a method by oral administration. That is, the dosage form of a quinoline carboxamide derivative or a salt thereof of the present invention is not particularly limited, and includes those that are orally or parenterally administered. Examples of oral preparations include oral solid preparations (e.g. tablets, coated tablets, granular tablets, powders and capsules), and oral liquid preparations (e.g. oral solutions, syrups and elixirs). Examples of the parenteral administration include injection, and intranasal, transtracheal, muscular and transdermal administration, and examples of parenteral preparations include injections (intravenous injections such as drips, hypodermic injections, intramuscular injections and intraperitoneal injections), ointments, patches, gels, creams, powders for external use, sprays and inhalational dusting powders.

A preparation comprising a quinoline carboxamide derivative or a salt thereof in the present invention can be normally prepared by a known method with the use of a pharmacologically acceptable carrier. Examples of the carrier include various carriers which are commonly used in usual agents, for example excipients, binders, disintegrants, lubricants, diluents, solubilizing agents, suspending agents, tonicity agents, pH adjusters, buffers, stabilizers, colorants, flavor improving agents and odor improving agents.

Examples of the excipient include lactose, sucrose, sodium chloride, glucose, maltose, mannitol, erythritol, xylitol, maltitol, inositol, dextran, sorbitol, albumin, urea, starch, calcium carbonate, kaolin, crystalline cellulose, silicic acid, methylcellulose, glycerin, sodium alginate, gum arabic and mixtures thereof. Examples of the lubricant include purified talc, stearates, borax, polyethylene glycol and mixtures thereof. Examples of the binder include simply syrup, glucose solution, starch solution, gelatin solution, polyvinyl alcohol, polyvinyl ether, polyvinyl pyrrolidone, carboxymethylcellulose, shellac, methylcellulose, ethylcellulose, water, ethanol, potassium phosphate and mixtures thereof. Examples of the disintegrant include dry starch, sodium alginate, powdered agar, powdered laminaran, sodium hydrogencarbonate, calcium carbonate, polyoxyethylene sorbitan fatty acid esters, sodium lauryl sulfate, monoglyceride stearate, starch, lactose and mixtures thereof. Examples of the diluent include water, ethyl alcohol, macrogol, propylene glycol, ethoxylated isostearyl alcohol, polyoxylated isostearyl alcohol, polyoxyethylene sorbitan fatty acid esters and mixtures thereof. Examples of the stabilizer include sodium pyrosulfite, ethylenediaminetetraacetic acid, thioglycolic acid, thiolactic acid and mixtures thereof. Examples of the tonicity agent include sodium chloride, boric acid, glucose, glycerin and mixtures thereof. Examples of the pH adjuster and the buffer include sodium citrate, citric acid, sodium acetate, sodium phosphate and mixtures thereof. Examples of the soothing agent include procaine hydrochloride, lidocaine hydrochloride and mixtures thereof.

The amount of a quinoline carboxamide derivative or a salt thereof in the present invention blended in the preparation can be appropriately set, and in general, the amount of the quinoline carboxamide derivative or a salt thereof according to the present invention in the preparation is from 0.001 to 5,000 mg, preferably from 0.1 to 1,000 mg, more preferably from 1 to 500 mg.

The dosage amount of a quinoline carboxamide derivative or a salt thereof in the present invention is not limited as long as the quinoline carboxamide derivative can exhibit an antitumor effect to effectively treat cancer, and is appropriately set according to the age of a patient, the cancer species, the disease stage, whether metastasis occurs or not, the treatment history, whether other antitumor agents are present or not, or the like. The amount of a quinoline carboxamide derivative of formula (I) or a salt thereof is preferably from 0.001 to 5,000 mg/day, preferably from 0.1 to 1,000 mg/day, more preferably 1 to 500 mg.

In the present invention, the dosage form of the immune checkpoint inhibitor is arbitrary, and intravenous administration is suitable. The dosage amount of the immune checkpoint inhibitor each time is not particularly limited, and can be appropriately set according to the carcinoma, the side effect or the like. The dosage amount each time is preferably from 0.001 to 5,000 mg, more preferably from 0.1 to 2,000 mg, especially preferably from 1 to 1,200 mg.

The above-described dose of the immune checkpoint inhibitor may be administered at a time, or in several parts (e.g. two to four parts) in a sustained manner. The administration interval is not limited, and the administration may be performed every day, every few days, every other week, every few weeks, every other month or every few months. For example, administration is performed once a week, once every two weeks, once every three weeks, once every four weeks, once a month, once every three months, or once every three to six months.

The schedule of administration of the antitumor agent of the present invention can be appropriately set according to the schedule of administration of each immune checkpoint inhibitor. For example, a quinoline carboxamide derivative or a salt thereof is administered every day from day 1, and each checkpoint inhibitor is administered on day 3, followed by appropriately conforming to the regimen of each immune checkpoint inhibitor; a quinoline carboxamide derivative or a salt thereof is administered every day from day 1, and each checkpoint inhibitor is administered on day 8, followed by appropriately conforming to the regimen of each immune checkpoint inhibitor; a quinoline carboxamide derivative or a salt thereof is administered every day from day 1, and each checkpoint inhibitor is administered on day 15, followed by appropriately conforming to the regimen of each immune checkpoint inhibitor; a quinoline carboxamide derivative or a salt thereof is administered every day from day 1, and each checkpoint inhibitor is administered on day 15, followed by appropriately conforming to the regimen of each immune checkpoint inhibitor; or a quinoline carboxamide derivative or a salt thereof is administered every day from day 1, and each checkpoint inhibitor is administered on day 28, followed by appropriately conforming to the regimen of each immune checkpoint inhibitor.

For the antitumor agent of the present invention, a quinoline carboxamide derivative or a salt thereof and an immune checkpoint inhibitor are administered in combination to a patient, and the means for providing both the agents is not particularly limited. That is, preparations comprising the agents may be separately provided together with a product document indicating a regimen, a dose and the like in combined use, etc., or each preparation may be packaged in a form suitable for combined administration each time.

### Examples

<Agents Used>

### (1) Quinoline carboxamide derivative

N-[5-(2-furyl)-1,3,4-oxadiazol-2-yl]-2-phenyl-6-trifluoromethoxy-4-quinoline carboxamide (hereinafter, referred to as "STX-1159") represented by the following formula was synthesized in accordance with the method described in Patent Literature 1.

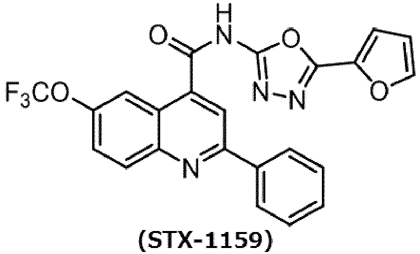

### (2) Immune checkpoint inhibitor

An anti-PD-1 antibody (anti-mouse PD-1 antibody) was purchased from Biolegend.

### Example 1: Antitumor effect of combined administration of quinoline carboxamide derivative and immune checkpoint inhibitor (1)

### 1. Method

### (1) Transplantation of tumor in mouse

Six-week-old female BALB/c mice (72 mice) were purchased from Japan SLC, Inc., conditioned and raised for 5 days in University of Shizuoka, Experimental Animal Center, and then used for experiments.

Hair on a back part and ventral parts of the BALB/c mouse was shaved, and 1 × 10⁶ mouse bowel cancer cell line CT26 cells (ATCCs) were subcutaneously transplanted in each of the left and right ventral parts (day 0). Five days after the transplantation, the longer diameter (mm) and the shorter diameter (mm) of the tumor were measured using a digital caliper (A&D Company, Limited), and a tumor volume was calculated. The expression for calculation of the tumor volume was as follows: tumor volume (mm³) = 0.5 × (longer diameter mm) × (shorter diameter mm)².

An average of the tumor volumes on the left and the right was calculated for each individual, and on the basis of the thus-obtained value, the mice were divided into eight groups (nine mice in each group) in such a manner that the variations in tumor volume between individuals in the groups were the same.

### (2) Administration of agent

STX-1159 was orally administered at 40 mg/kg continuously for five days. Specifically, a suspension liquid was prepared at 4 mg/mL using a 0.5% methylcellulose solution (Wako), and orally administered at 0.01 mL per g of the body weight of the BALB/c mouse using a metallic tube (φ 0.7 × 50 mm, Natsume Seisakusho Co., Ltd.). The schedule of administration was set to a period before administration of the anti-mouse PD-1 antibody (from day 5 to day 9), a period identical to that of administration of the anti-mouse PD-1 antibody (from day 12 to day 16) or after administration of the anti-mouse PD-1 antibody (from day 19 to day 23). For individuals in a group to which STX-1159 was not administered, the 0.5% methylcellulose solution was orally administered.

The anti-mouse PD-1 antibody was intraperitoneally administered at 50 µg/mouse three times every other day. Specifically, anti-mouse CD279 (PD-1) (Biolegend) was diluted to 250 µg/mL with PBS (Wako), and intraperitoneally administered at 200 µL/BALB/c mouse with a 29G needle-tipped syringe (BD). The schedule of administration was set to day 12, day 14 and day 16. For individuals in a group to which the anti-mouse PD-1 antibody was not administered, 200 µL of PBS was intraperitoneally administered. Specific groupings are described below.

1) Control
2) PD-1 Ab (50 µg/mouse i.p. days 12, 14 and 16)
3) STX-1159 × five times (40 mg/kg p.o. days 5 to 9)
4) STX-1159 × five times (40 mg/kg p.o. days 12 to 16)
5) STX-1159 × five times (40 mg/kg p.o. days 19 to 23)
6) STX-1159 × five times (40 mg/kg p.o. days 5 to 9) + PD-1 Ab (50 µg/mouse i.p. days 12, 14 and 16)
7) STX-1159 × five times (40 mg/kg p.o. days 12 to 16) + PD-1 Ab (50 µg/mouse i.p. days 12, 14 and 16)
8) STX-1159 × five times (40 mg/kg p.o. days 19 to 23) + PD-1 Ab (50 µg/mouse i.p. days 12, 14 and 16)

### (3) Evaluation item

The evaluation item was an average of the tumor volumes on the left and the right in each individual. From day 5, the longer diameter (mm) and the shorter diameter (mm) of the tumor were measured at an interval of one day or two days using a digital caliper. The expression for calculation of the tumor volume was as follows: tumor volume (mm³) = 0.5 × (longer diameter mm) × (shorter diameter mm)². An average of the tumor volumes on the left and the right was calculated for each individual.

### (4) Evaluation index

For evaluating the effect of administration of the agent on the tumor volume, a t-test was conducted on the evaluation items for agent-administration groups versus the evaluation item for the control group. When p < 0.05 or p < 0.01 was met, it was considered that there was a significant difference, and administration of the agent was determined to have an effect on the tumor volume.

### 2. Results

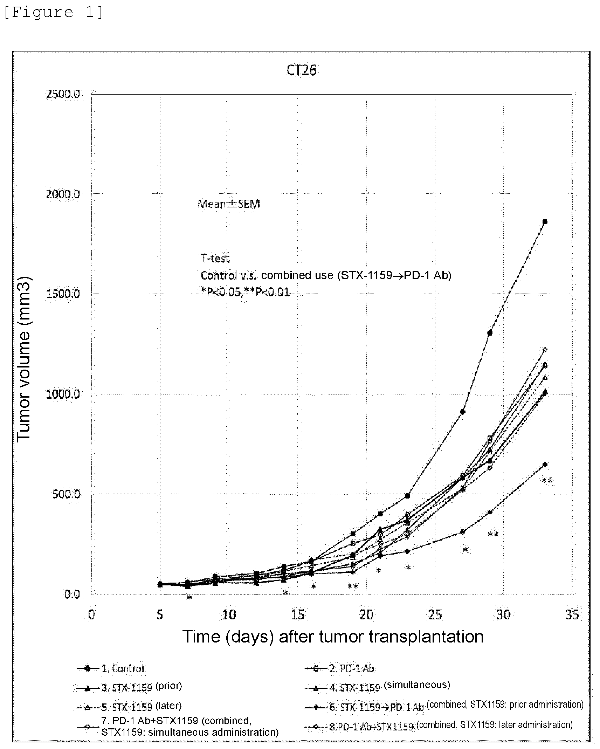

Figure 1 shows the results.

In the group to which the quinoline carboxamide derivative was administered in advance (STX-1159 (days 5 to 9) + PD-1 Ab (days 12, 14 and 16)), a much higher antitumor effect was observed than in the simultaneous administration group (STX-1159 (days 12 to 16) + PD-1 Ab (days 12, 14 and 16)) and the later administration group (STX-1159 (days 19 to 23) + PD-1 Ab (days 12, 14 and 16)). In particular, in the simultaneous administration group (STX-1159 (days 12 to 16) + PD-1 Ab (days 12, 14 and 16)), the antitumor effect was lower than that in each of the PD-1 Ab single-agent group (days 12, 14 and 16), the STX-1159 single-agent group (days 5 to 9), the STX-1159 single-agent group (days 12 to 16) and the STX-1159 single-agent group (days 19 to 23) which are single-agent administration groups.

### Example 2: Antitumor effect of combined administration of quinoline carboxamide derivative and immune checkpoint inhibitor (2)

### 1. Method

### (1) Transplantation of tumor in mouse

Six-week-old female BALB/c mice were purchased from Japan SLC, Inc., conditioned and raised for about a week in University of Shizuoka, Experimental Animal Center, and then used for experiments.

Hair on a back part and ventral parts of the BALB/c mouse was shaved, and 1 × 10⁶ mouse bowel cancer cell line CT26 cells (ATCCs) were subcutaneously transplanted in each of the left and right ventral parts (day 0). Six days after the transplantation, the longer diameter (mm) and the shorter diameter (mm) of the tumor were measured using a digital caliper (A&D Company, Limited), and a tumor volume was calculated. The expression for calculation of the tumor volume was as follows: tumor volume (mm³) = 0.5 × (longer diameter mm) × (shorter diameter mm)². An average of the tumor volumes on the left and the right was calculated for each individual, and on the basis of the thus-obtained value, the mice were divided into groups in such a manner that the variations in tumor volume between individuals in the groups were the same.

### (2) Administration of agent

STX-1159 was orally administered at 40 mg/kg three times every other day. Specifically, a suspension liquid was prepared at 4 mg/mL using a 0.5% methylcellulose solution (Wako), and orally administered at 0.01 mL per g of the body weight of the BALB/c mouse using a metallic tube (φ 0.7 × 50 mm, Natsume Seisakusho Co., Ltd.). The schedule of administration was set to day 6, day 8 and day 10. For individuals in a group to which STX-1159 was not administered, the 0.5% methylcellulose solution was orally administered.

The anti-mouse PD-1 antibody was intraperitoneally administered at 50 µg/mouse three times every other day or every two days. Specifically, anti-mouse CD279 (PD-1) (Biolegend) was diluted to 250 µg/mL with PBS (Wako), and intraperitoneally administered at 200 µL/BALB/c mouse with a 29G needle-tipped syringe (BD). The schedule of administration was set to day 8, day 10 and day 13. For individuals in a group to which the anti-mouse PD-1 antibody was not administered, 200 µL of PBS was intraperitoneally administered. Specific groupings are described below.
1) Control
2) STX-1159 × three times (40 mg/kg p.o. days 6, 8 and 10)
3) PD-1 Ab (50 µg/mouse i.p. days 8, 10 and 13)
4) STX-1159 × three times (40 mg/kg p.o. days 6, 8 and 10) + PD-1 Ab (50 µg/mouse i.p. days 8, 10 and 13)

### (3) Evaluation item

The evaluation item was an individual tumor volume. From day 6, the longer diameter (mm) and the shorter diameter (mm) of the tumor were measured at an interval of one to four days using a digital caliper. The expression for calculation of the tumor volume was as follows: tumor volume (mm³) = 0.5 × (longer diameter mm) × (shorter diameter mm)².

### (4) Evaluation index

For evaluating the effect of administration of the agent on the tumor volume, a t-test was conducted on the evaluation items for agent-administration groups versus the evaluation item for the control group. When p < 0.05 was met, it was considered that there was a significant difference, and administration of the agent was determined to have an effect on the tumor volume.

### 2. Results

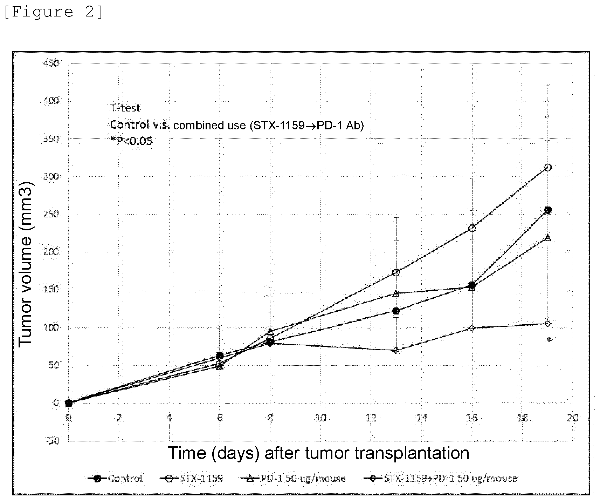

Figure 2 shows the results.

In the group of combined administration of a quinoline carboxamide derivative and an immune checkpoint inhibitor in which the quinoline carboxamide derivative was administered in advance (STX-1159 (days 6, 8 and 10) + PD-1 Ab (days 8, 10 and 13)), a much higher antitumor effect was observed than in the group of administration of a quinoline carboxamide derivative or an immune checkpoint inhibitor alone.

### Example 3: Antitumor effect of combined administration of quinoline carboxamide derivative and immune checkpoint inhibitor (3)

### 1. Method

### (1) Transplantation of tumor in mouse

Six-week-old female BALB/c mice were purchased from Japan SLC, Inc., conditioned and raised for about a week in University of Shizuoka, Experimental Animal Center, and then used for experiments.

Hair on a back part and ventral parts of the BALB/c mouse was shaved, and 1 × 10⁶ mouse fibrosarcoma CMS5a cells were subcutaneously transplanted in each of the left and right ventral parts (day 0). Five days after the transplantation, the longer diameter (mm) and the shorter diameter (mm) of the tumor were measured using a digital caliper (A&D Company, Limited), and a tumor volume was calculated. The expression for calculation of the tumor volume was as follows: tumor volume (mm³) = 0.5 × (longer diameter mm) × (shorter diameter mm)². An average of the tumor volumes on the left and the right was calculated for each individual, and on the basis of the thus-obtained value, the mice were divided into groups in such a manner that the variations in tumor volume between individuals in the groups were the same.

### (2) Administration of agent

STX-1159 was orally administered at 40 mg/kg continuously for five days. Specifically, a suspension liquid was prepared at 4 mg/mL using a 0.5% methylcellulose solution (Wako), and orally administered at 0.01 mL per g of the body weight of the BALB/c mouse using a metallic tube (φ 0.7 × 50 mm, Natsume Seisakusho Co., Ltd.). The schedule of administration was set to a period before administration of the anti-mouse PD-1 antibody (from day 5 to day 9). For individuals in a group to which STX-1159 was not administered, the 0.5% methylcellulose solution was orally administered.

The anti-mouse PD-1 antibody was intraperitoneally administered at 200 µg/mouse three times every other day. Specifically, anti-mouse CD279 (PD-1) (Biolegend) was diluted to 1 mg/mL with PBS (Wako), and intraperitoneally administered at 200 µL/BALB/c mouse with a 29G needle-tipped syringe (BD). The schedule of administration was set to day 12, day 14 and day 16. For individuals in a group to which the anti-mouse PD-1 antibody was not administered, 200 µL of PBS was intraperitoneally administered. Specific groupings are described below.
1) Control
2) PD-1 Ab (200 µg/mouse i.p. days 12, 14 and 16)
3) STX-1159 × five times (40 mg/kg p.o. days 5 to 9)
4) STX-1159 × five times (40 mg/kg p.o. days 5 to 9) + PD-1 Ab (200 µg/mouse i.p. days 12, 14 and 16)

### (3) Evaluation item

The evaluation item was an individual tumor volume. From day 5, the longer diameter (mm) and the shorter diameter (mm) of the tumor were measured at an interval of one or two days using a digital caliper.

The expression for calculation of the tumor volume was as follows: tumor volume (mm³) = 0.5 × (longer diameter mm) × (shorter diameter mm)².

### (4) Evaluation index

For evaluating the effect of administration of the agent on the tumor volume, a t-test was conducted on the evaluation items for agent-administration groups versus the evaluation item for the control group. When p < 0.05 or p < 0.01 was met, it was considered that there was a significant difference, and administration of the agent was determined to have an effect on the tumor volume.

### 2. Results

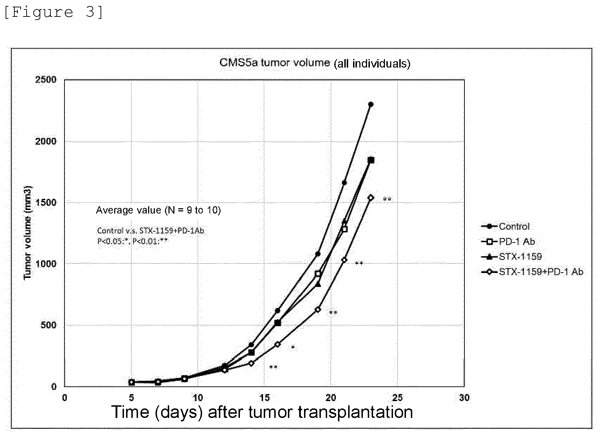

Figure 3 shows the results.

The group of combined administration of a quinoline carboxamide derivative and an immune checkpoint inhibitor in which the quinoline carboxamide derivative was administered in advance (STX-1159 (days 5 to 9) + PD-1 Ab (day 12, day 14 and day 16)) showed more significant tumor shrinkage over the control group. The group of administration of PD-1 Ab alone did not show more significant tumor shrinkage over the control group, and it was found that even for CMS5a resistant to an immune checkpoint inhibitor, an antitumor effect was obtained by administering a quinoline carboxamide derivative in combination with an immune checkpoint inhibitor.

## Claims

1. An antitumor agent comprising a quinoline carboxamide derivative of formula (I) below or a salt thereof as an active ingredient, the antitumor agent being administered in combination with an immune checkpoint inhibitor, wherein the antitumor agent is administered before the administration of the immune checkpoint inhibitor: 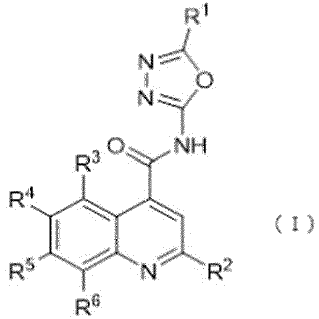 wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

2. An antitumor agent comprising a quinoline carboxamide derivative of formula (I) below or a salt thereof and an immune checkpoint inhibitor which are administered in combination, wherein the quinoline carboxamide derivative or a salt thereof is administered before the administration of the immune checkpoint inhibitor: 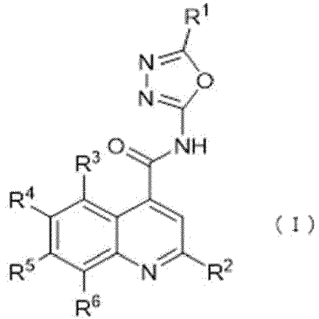 wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

3. The antitumor agent according to claim 1 or 2, wherein the administration of the quinoline carboxamide derivative or a salt thereof is started one or more days before the administration of the immune checkpoint inhibitor.

4. The antitumor agent according to any one of claims 1 to 3, wherein the administration of the quinoline carboxamide derivative or a salt thereof is started two to seven days before the administration of the immune checkpoint inhibitor.

5. The antitumor agent according to any one of claims 1 to 4, wherein the route of administration of the quinoline carboxamide derivative or a salt thereof is oral administration.

6. The antitumor agent according to any one of claims 1 to 5, wherein R¹ and R² in formula (I) are the same or different, and each represent a substituted or unsubstituted aryl group or a substituted or unsubstituted aromatic heterocyclic group.

7. The antitumor agent according to claim 6, wherein at least one of the groups of R³, R⁴, R⁵ and R⁶ in formula (I) is a group other than a hydrogen atom.

8. The antitumor agent according to any one of claims 1 to 7, wherein in R¹ and R² in formula (I), the aryl group is a phenyl group and the aromatic heterocyclic group is a furyl group.

9. The antitumor agent according to any one of claims 1 to 7, wherein in formula (I), R¹ is a furyl group and R² is a substituted or unsubstituted phenyl group.

10. The antitumor agent according to any one of claims 1 to 9, wherein at least one of the groups of R³, R⁴, R⁵ and R⁶ is a trifluoromethoxy group.

11. The antitumor agent according to any one of claims 1 to 10, wherein R⁴ is a trifluoromethoxy group.

12. The antitumor agent according to any one of claims 1 to 11, wherein the quinoline carboxamide derivative of formula (I) is N-[5-(2-furyl)-1,3,4-oxadiazol-2-yl]-2-phenyl-6-trifluoromethoxy-4-quinolinecarboxamide.

13. The antitumor agent according to any one of claims 1 to 12, wherein the immune checkpoint inhibitor is a substance which suppresses a checkpoint function by acting on an immune checkpoint molecule selected from the group consisting of CTLA-4, PD-1, PD-L1, PD-L2, LAG-3, TIM3, BTLA, B7H3, B7H4, 2B4, CD160, A2aR, KIR, VISTA and TIGIT.

14. The antitumor agent according to any one of claims 1 to 12, wherein the immune checkpoint inhibitor is a PD-1 pathway inhibitor.

15. The antitumor agent according to claim 14, wherein the PD-1 pathway inhibitor is an anti-PD-1 antibody or an anti-PD-Ll antibody.

16. The antitumor agent according to any one of claims 1 to 15, wherein the cancer is one or more selected from the group consisting of non-small cell lung cancer, renal cell cancer, Hodgkin lymphoma, head and neck cancer, stomach cancer, malignant pleural mesothelioma, esophagus cancer, gastroesophageal cancer, small cell cancer, glioblastoma, urothelial cancer, muscle invasion urothelial cancer, urinary bladder cancer, non-muscle invasion urinary bladder cancer, bowel cancer, pancreas cancer, prostate cancer, Merkel cell carcinoma, thyroid gland cancer, hepatocyte cancer, breast cancer, ovary cancer, uterine body cancer, uterine cervical cancer, soft tissue sarcoma, virus-positive/negative solid cancer, central nerve-origin lymphoma/testis-origin lymphoma, MSI-High solid cancer, melanoma and leukemia.

17. Use of a quinoline carboxamide derivative of formula (I) below or a salt thereof for producing an antitumor agent which is administered in combination with an immune checkpoint inhibitor, the antitumor agent being administered before the administration of the immune checkpoint inhibitor: 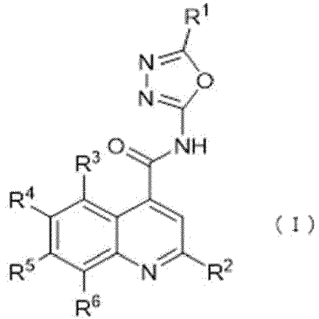 wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

18. A quinoline carboxamide derivative of formula (I) below or a salt thereof which is used in combination with an immune checkpoint inhibitor for treatment of cancer and administered before the administration of the immune checkpoint inhibitor: 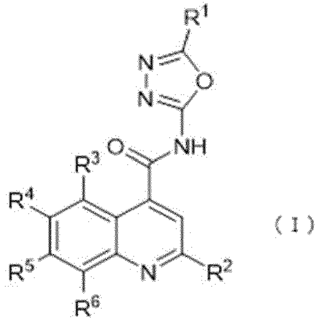 wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).

19. A method for treating cancer, comprising administering an effective amount of a quinoline carboxamide derivative of formula (I) below or a salt thereof and an immune checkpoint inhibitor, wherein the quinoline carboxamide derivative or a salt thereof is administered before the administration of the immune checkpoint inhibitor: 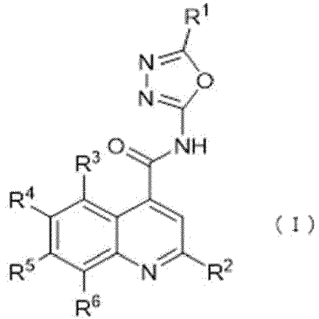 wherein R¹, R², R³, R⁴, R⁵ and R⁶ are the same or different, and each represent a hydrogen atom, a substituted or unsubstituted aryl group, a substituted or unsubstituted aromatic heterocyclic group, COOR⁷ (wherein R⁷ represents a substituted or unsubstituted alkyl group), or OR⁸ (wherein R⁸ represents a substituted or unsubstituted alkyl group).
